# EUROPEAN PATENT SPECIFICATION

(11) **EP 0 344 907 B1**
(45) Date of publication and mention of the grant of the patent: **16.09.1998**
(21) Application number: 89304254.9
(22) Date of filing: 27.04.1989
(51) Int. Cl.: A61M 25/00, F16J 15/00

(54) **Self-sealing guidewire and catheter introducer**
Selbstdichtender Führungsdraht und Vorrichtung zum Einführen eines Katheters
Guide métallique aut-étanche et cathéter introducteur

(30) Priority: 02.06.1988 US 201538
(43) Date of publication of application: 06.12.1989
(73) Proprietor: C.R. BARD, INC., Murray Hill New Jersey 07974 (US)
(72) Inventor: Sylvanowicz, John T., Andover, MA 01810 (US); Bourne, George W., IV, North Chelmsford, MA 01863 (US)
(74) Representative: Marsh, Roy David

(56) References cited:
- FR-A- 2 049 513
- US-A- 4 424 833
- US-A- 4 430 081
- US-A- 4 626 245

## Description

This invention relates to improvements in hemostasis catheter introducers used in the insertion and removal of catheters and guidewires from a patient's blood vessel. More particularly, the invention relates to an introducer having a self-sealing gasket which prevents back bleeding with inserted devices such as relatively slender guidewires as well as more substantial diameter catheters. An introducer corresponding to the pre-characterising part of claim 1 below is disclosed in US-A-4 424 833.

US Patent No. 4.424.833 discloses an introducer for insertion into a patient's blood vessel comprising a housing having an introducer sheath extending from an end of the housing, the sheath being adapted to be inserted into a patient's blood vessel, the housing having an opening at the other end, a self-sealing one piece gasket mounted in the housing and being exposed at the opening, the gasket having a central hole in its outwardly facing surface forming a wall which contacts a guidewire or catheter, the inwardly facing surface of the gasket being formed with a plurality of radially extending slits which define a plurality of flaps normally closing the hole.

The self-sealing gasket of this catheter introducer is adapted to maintain a seal and prevent back bleeding both when the catheter is in place through the introducer as well as when the catheter is removed. It also includes a side entry port through which liquids may be introduced or through which the device may be aspirated. The device is effective to provide a self-sealing gasket that maintains its seal over a relatively wide range of pressures to include not only the normal positive pressure of the patient's blood but also the reduced pressure which results from aspirations through the side port. The device also displays the desirable feature that its self-sealing function is achieved without unduly inhibiting axial and rotary movement of the catheter as it passes through the seal.

Although the foregoing device maintains an effective seal with a catheter passing through it as well as when the catheter is not present, it is somewhat limited in the range of diameters of devices that can be passed through it while maintaining a seal. In particular, guidewires, which typically are used in association with catheters, are of significantly smaller diameter than the catheters and when passed through the gasket, do not maintain an effective seal with the gasket. The guidewires, however, are sufficiently large to break the seal which permits blood to leak out of the proximal end of the device. Therefore, it would be desirable to provide a device having a self-sealing structure that is effective to provide a seal when a catheter as well as when a guidewire extends through the introducer. Moreover, such a device also should be effective to maintain the seal when neither the guidewire nor catheter are in place as well as during aspiration and over a relatively wide range of pressures. Further, such a device should not unduly inhibit axial as well as rotary movement of the catheter or the guidewire.

It is among the general objects of the invention to provide a catheter introducer with an improved self-sealing gasket.

A further object of the invention is to provide a catheter introducer having a self-sealing gasket adapted to effect a seal with a guidewire as well as with a catheter but without unduly restricting the ability of the guidewire or catheter to be manipulated therethrough.

The present invention overcomes the problems of the prior art device by providing a catheter introducer with the technical features recited in claim 1 below.

In a preferred embodiment of the invention, the self-sealing gasket is molded in a single piece from a resilient material, preferably from a high durometer elastomer to have a predetermined thickness. The outer half thickness of the gasket has a central hole, slightly smaller in diameter than the diameter of the guidewire that will be received in the device, and which will form a seal about the guidewire. The inner half thickness of the gasket is provided with a plurality of radially extending slots which extend to a depth of the middle of the thickness of the gasket. The combination of the slits and the central hole define an arrangement which is effective to make a seal against a guidewire as well as with a much larger diameter catheter and without unduly inhibiting movement of either of the guidewire or catheter through the gasket. The arrangement defines a number of flaps which normally close the central hole in the absence of a guidewire or a catheter. When a guidewire is inserted into the device, the flaps spread apart and a seal is made against the guidewire by the periphery of the central hole in the outer face of the gasket. When a larger diameter catheter is inserted through the device, the resilience of the gasket enables the hole to enlarge. Restriction on the movement of the catheter, however, is minimized because the inner or distal half thickness of the gasket, being slit, readily yields and presents no significant constricting force about the catheter. The constricting, sealing force about the catheter is provided only by the outer half thickness of the gasket.

The foregoing and other objects and advantages of the invention will be appreciated more fully from the following further description thereof by way of example only, with reference to the accompanying drawings, in which:
FIGURE 1 is an illustration of the invention as embodied in the housing of catheter introducer broken away in section to illustrate the internal structure of the device;
FIGURE 2 is an enlarged sectional illustration taken along the line 2-2 of the one-piece self-sealing valve element of the present invention shown in Figure 3;
FIGURE 3 is an illustration of the self-sealing valve member as viewed from the left (proximal) face of Figure 2;
FIGURE 4 is an illustration of the device having a catheter extending through the introducer with the gasket deformed to receive the catheter in self-sealing configuration; and
FIGURE 5 is an illustration of the device similar to Figure 4 with a guidewire extending therethrough.

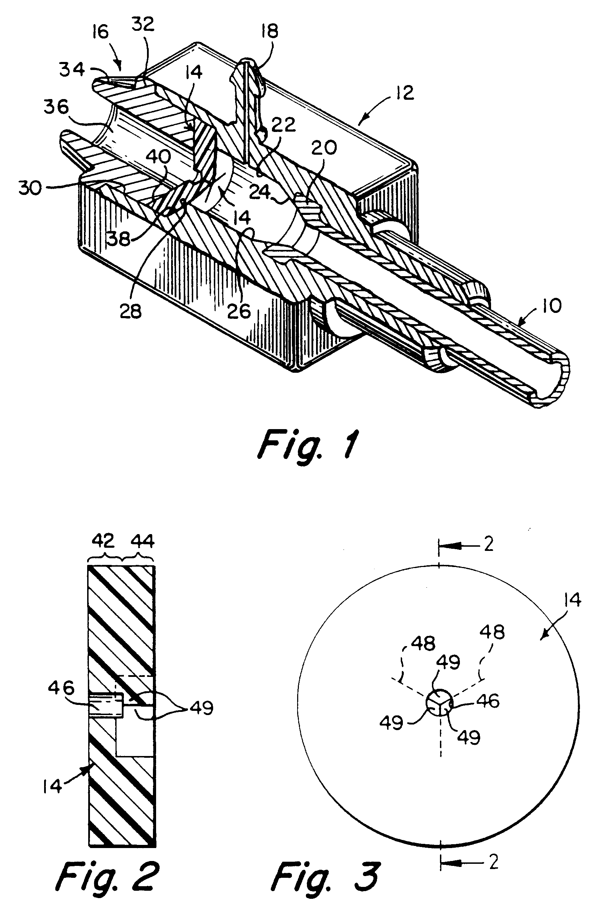

Figure 1 shows a catheter introducer incorporating the present invention. The introducer includes an introducer tube 10 which is connected to and extends from a housing 12. The one-piece molded self-sealing gasket, indicated generally by the reference character 14 is mounted in the valve housing 12 as described more fully below. The gasket 14 is retained firmly in place in the housing 12 by an end cap 16. The housing 12 preferably is provided with a side port 18.

The introducer tube 10 may be formed from a fluorinated plastic and is formed separately with an enlarged head portion 20. The housing 12 preferably is molded directly onto the headed end of the introducer tube 10 and is locked securely and integrally with the introducer tube 10 by engagement with the head portion 20. The housing 12 is formed with a hollow interior 22 which forms a forwardly tapering confiquration indicated at 24, which merges smoothly with the tapering inlet and of the head portion 20 of the introducer tube 10. The side port 18 is molded integrally with the housing to provide a means to communicate directly with the hollow interior 22 of the housing 12.

The hollow interior 22 of the housing 12 defines a cylindrical bore 26 which terminates in an enlarged diameter shoulder 28. The shoulder 28 terminates in an enlarged diameter outer bore 30 which receives the end cap 16. The end cap 16 has an inner portion dimensioned to fit snugly within the outer bore 30. The cap 16 includes an outer peripheral collar 32 which engages the outer end of the housing to determine and limit precisely the extent to which the inner end of the cap 16 extends into the outer bore 30. The outer end of the end cap 16 may include an extension 34 and a tapered inlet opening 36 may be formed through the end cap. The inlet 36 is tapered to enable a conventional luer connector to be attached, if desired.

As shown in FIG. 1, the self-sealing gasket 14 is retained between the end cap 16 and the shoulder 28 of the housing. The shoulder 28 preferably is provided with a circular ridge 38 and an identical ridge 40 is formed on the facing inner surface of the inner end of the end cap 16. When the end cap 16 is fully seated, as determined by engagement of the collar 32 with the end of the housing 12, the ridges 38, 40 engage and effect a firm grip on the gasket 14.

As shown in enlarged detail in FIGS. 2-5, the gasket 14 may be considered as having an outer half thickness 42 and an inner half thickness 44. The outer half thickness 42 is formed with a central hole 46 which extends only to a depth of about the half thickness of the gasket 14. The inner half thickness of the gasket is formed with a plurality (preferably three) radially extending slits 48 which extend slightly greater than the half thickness of the gasket 14. The slits 48 define three approximately triangularly shaped flaps 49 that overlap the central hole 46. The diameter of the central hole 46 is selected so that it will effect a seal with the guidewires with which the device may be used. The radially extending slits 48, on the inner face of the gasket 14 should extend radially a distance that is slightly greater than the maximum diameter of catheter with which the device is to be used. By way of example, for a maximum catheter diameter of the order of 2.64mm (0.104") (8 French) the disk-shaped gasket may be 7.87mm (0.310") in diameter and 1.78mm (0.070") thick. The central hole 46 may be approximately 0.74mm (0.029") diameter, that diameter being adapted to effect a seal with conventional size guidewires of the order of 0.89mm (0.035") to about 0.97mm (0.038"). The central hole depth is about 0.89mm (0.035").

The length of the radial slits is important in that if the slits 48 are too long, the flaps 49 will tend to fold in a manner that increases the drag of the guidewire and/or catheter but if the slits are too short, the resulting flaps are too stiff and will unduly restrict movement of the guidewire and catheter. We have found that for a gasket as described above a radial length of about 1.90mm (0.075") for the slits 48 is ideal to accept catheters of 5 French to 9 French size.

It is very desirable that the gasket 14 be molded from a comparatively hard, low friction resilient material, such as a high durometer silicone rubber having a hardness of at least 35 A and preferably between 35 A and 60 A and most preferably near 50 A on the Shore A Scale. The gasket preferably is lubricated with a silicone oil.

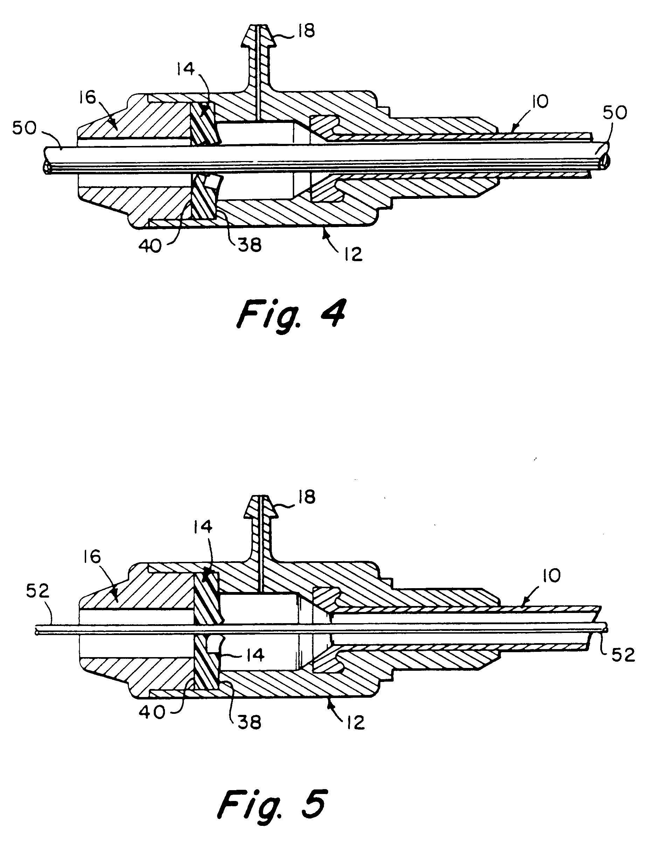

FIG. 4 illustrates the configuration of the gasket 14 with a catheter 50 extending therethrough. As can be seen, the catheter 50 forces enlargement of the central hole 46 in the outer half thickness 42 of the gasket 14, and it is that portion of the gasket that provides the constricting and sealing force. That force is not too great to adversely impair catheter movement because it is affected only by half of the thickness of the gasket, the inner half thickness of the gasket 44 being slit and providing no substantial constricting force.

FIG. 5 illustrates the manner in which the gasket 14 effects a seal about a guidewire 52. The seal is effected by the periphery of the central hole 46 which is slightly smaller in diameter than the diameter of the guidewire 51. The flaps 49 formed on the inner half thickness 44 of the gasket 14 and defined by the portion of the radial slits 48 that overlap the central hole 46, separate as shown, to permit free movement of the guidewire 52. As mentioned above, the stiffness of the material, as measured by the Shore A Scale, is no less than about 35 A and preferably is of the order of 50 A. When both the catheter and the guidewire are removed, the flaps 49 close thereby providing a seal to prevent back bleeding.

In using the device, the introducer tube 10 is inserted into a patient's blood vessel, typically percutaneously. Once the introducer is in place it may be sutured to the patient's skin to secure it. Thereafter, guidewires and catheters may be introduced through the self-sealing gasket 14 in housing 12 and catheter changes may be made as desired. When using angiographic procedures or other surgical techniques in which the physician must be able to feel obstructions to the advancing catheter or guidewire tip by feeling resistance at the proximal end of the catheter, the resistance offered by the valve 14 of the present invention does not significantly impair the physician's feel. The side port 18, of course, may be used in the manner in which side ports are normally used, such as to infuse medicine, intravenous nourishment or to take blood pressure measurements. The side port may be aspirated to withdraw blood samples if desired, and in aspiration, the device is quite satisfactory.

The gasket is easy to make and assemble in the introducer body.

It should be understood that the foregoing description of the invention is intended merely to be illustrative thereof and that other modifications and embodiments may be apparent to those skilled in the art without departing from the scope of the invention definded in the claims.

## Claims

1. An introducer adapted to be inserted into a patent's blood vessel comprising:
a housing (12) having an introducer sheath (10) extending from an end of the housing, the sheath being adapted to be inserted into a patient's blood vessel, the housing having an opening (36) at the other end;
a self-sealing one piece gasket (14) mounted in the housing and being exposed at the opening, the gasket having a central hole (46) in its outwardly facing surface, the hole depth being that of an outer half thickness (42) of the gasket, the inwardly facing surface of the gasket bounding an inner half thickness (44) of the gasket which is formed with at least three radially extending slits (48), each of which extends from the inwardly facing surface to the base of the hole, and radially outwardly from a central region of the slits overlapping the central hole (46), the slits together defining a plurality of flaps (49) normally closing the hole;
the introducer being **characterized by**:
the radius of the central hole (46) at its base being substantially smaller than the length defined by each of the slits (48) in their direction of radial extent.

2. A device as claimed in claim 1 characterised in that the diameter of the central hole (46) is approximately 0.74mm (0.029") and the length of each radial slit (48) is approximately 1.90mm (0.075").

3. A device as claimed in claims 1 or 2 characterised in that the gasket (14) is formed from a moulded elastomeric material having a Shore A durometer of between about 35 A and 60 A.

4. A device as claimed in any preceding claim characterised in that the gasket (14) is approximately 1.18mm (0.070") thick.

5. A device as claimed in claim 1 characterised in that the housing includes an inner bore (26) and an outer bore (30), the outer bore being larger than the inner bore and defining a shoulder (28) at the juncture of the inner bore and the outer bore, the gasket (14) having a diameter corresponding substantially to that of the outer bore, and being secured in the housing by a cap (16) receivable in the outer bore of the housing to tightly compress the periphery of the gasket (14) against the shoulder (28).

## Patentansprüche

1. Einführvorrichtung, die dazu geeignet ist, in ein Blutgefäß eines Patienten eingeführt zu werden, umfassend:
ein Gehäuse (12) mit einer Einführumhüllung (10), die sich von einem Ende des Gehäuses erstreckt, wobei die Umhüllung dazu geeignet ist, in ein Blutgefäß eines Patienten eingeführt zu werden, und wobei das Gehäuse eine Öffnung (36) am anderen Ende besitzt;
eines selbstdichtende einstückige Dichtung (14), die in das Gehäuse eingesetzt ist und an der Öffnung freiliegt, wobei die Dichtung ein mittiges Loch (46) in ihrer nach außen gerichteten Oberfläche aufweist und die Tiefe des Loches jene der äußeren halben Dicke (42) der Dichtung ist und wobei die nach innen gerichtete Oberfläche der Dichtung eine innere Halbdicke (44) der Dichtung begrenzt, die mit zumindest drei sich in radialer Richtung erstreckenden Schlitzen (48) gebildet ist, von denen sich alle von der nach innen gerichteten Oberfläche zum Boden des Loches und radial nach außen von einem mittleren Bereich der Schlitze in Überlappung mit dem mittigen Loch (46) erstrecken, wobei die Schlitze zusammen eine Mehrzahl von Klappen (49) begrenzen, die normalerweise das Loch schließen;
wobei die Einführvorrichtung **dadurch gekennzeichnet ist, daß**
der Radius des mittigen Loches (46) an seinem Boden wesentlich kleiner als die Länge ist, die durch jeden der Schlitze (48) in seiner Richtung der radialen Erstreckung definiert ist.

2. Vorrichtung gemäß Anspruch 1, **dadurch gekennzeichnet, daß** der Durchmesser des mittigen Loches (46) etwa 0,74 mm (0,029") und die Länge jedes radialen Schlitzes (48) etwa 1,90 mm (0,075") ist.

3. Vorrichtung gemäß Anspruch 1 oder 2, **dadurch gekennzeichnet, daß** die Dichtung (14) aus einem gegossenen elastomerischen Material mit einem Shore A Durometer von etwa zwischen 35 A und 60 A gebildet ist.

4. Vorrichtung gemäß einem der vorhergehenden Ansprüche, **dadurch gekennzeichnet, daß** die Dichtung (14) etwa 1,18 mm (0,070") dick ist.

5. Vorrichtung gemäß Anspruch 1, **dadurch gekennzeichnet, daß** das Gehäuse eine innere Bohrung (26) und eine äußere Bohrung (30) aufweist, wobei die äußere Bohrung größer als die innere Bohrung ist und eine Schulter (28) an der Verbindung der inneren Bohrung und der äußeren Bohrung definiert, die Dichtung (14) einen Durchmesser besitzt, der im wesentlichen jenem der äußeren Bohrung entspricht und im Gehäuse durch eine Kappe (16) befestigt ist, die in der äußeren Bohrung des Gehäuses aufnehmbar ist, um fest den Umfang der Dichtung (14) gegen die Schulter (28) zu drücken.

## Revendications

1. Dispositif d'introduction à insérer dans un vaisseau sanguin d'un malade comprenant :
un boîtier (12) ayant un manchon introducteur (10) partant d'une extrémité du boîtier, le manchon étant conçu pour être inséré dans un vaisseau sanguin d'un malade, le boîtier ayant une ouverture (36) à l'autre extrémité;
un joint d'étanchéité auto-étanchéifiant en une seule pièce (14) monté dans le boîtier et exposé à l'ouverture, le joint d'étanchéité ayant un trou central (46) dans sa surface tournée vers l'extérieur, la profondeur du trou étant celle de la demi-épaisseur (42) du joint d'étanchéité, la surface tournée vers l'intérieur du joint d'étanchéité reliant une demi-épaisseur interne (44) du joint d'étanchéité qui est constitué d'au moins trois fentes (48) s'étendant radialement, dont chacune s'étend de la surface tournée vers l'intérieur vers la base du trou, et radialement vers l'extérieur à partir d'une région centrale des fentes recouvrant partiellement le trou central (46), les fentes définissant ensemble une pluralité de volets (44) fermant normalement le trou,
le dispositif introducteur étant caractérisé en ce que :
le rayon du trou central (46) à sa base est nettement plus faible que la longueur définie par chacune des fentes (48) dans la direction de leur étendue radiale.

2. Dispositif selon la revendication 1, caractérisé en ce que le diamètre du trou central (46) est d'environ 0,74 mm (0,029") et la longueur de chacune des fentes radiales (48) est d'environ 1,90 mm (0,075").

3. Dispositif selon les revendications 1 ou 2, caractérisé en ce que le joint d'étanchéité (14) est formé d'une matière élastomère moulée ayant une dureté Shore A comprise entre environ 35 A et 60 A.

4. Dispositif selon l'une quelconque des revendications précédentes, caractérisé en ce que le joint d'étanchéité (14) a environ 1,18 mm (0,070") d'épaisseur.

5. Dispositif selon la revendication 1, caractérisé en ce que le boîtier contient un trou interne (26) et un trou externe (30), le trou externe étant plus grand que le trou interne et définissant un rebord (28) à la jonction du trou interne et du trou externe, le joint d'étanchéité (14) ayant un diamètre correspondant pratiquement à celui du trou externe, et étant fixé dans le boîtier par un capuchon (16) pouvant se loger dans le trou externe du boîtier en comprimant fortement la périphérie du joint d'étanchéité (14) contre le rebord (28).
